# FASCICULE DE BREVET EUROPEEN

(11) **EP 2 595 567 B1**
(45) Date de publication et mention de la délivrance du brevet: **21.12.2016**
(21) Numéro de dépôt: 11743304.5
(22) Date de dépôt: 06.07.2011
(51) Int. Cl.: A61F 2/02

(54) **POCHE POUR FORMER UN ORGANE ARTIFICIEL IMPLANTABLE**
BEUTEL ZUR FORMUNG EINES IMPLANTIERBAREN KÜNSTLICHEN ORGANS
BAG FOR FORMING AN IMPLANTABLE ARTIFICIAL ORGAN

(30) Priorité: 22.07.2010 FR 1056004
(43) Date de publication de la demande: 29.05.2013
(73) Titulaire: STATICE MANUFACTURING, 25000 Besancon (FR)
(72) Inventeur: PIRANDA, Serge, F-25000 Besancon (FR)
(74) Mandataire: Novagraaf Technologies
(86) Numéro de dépôt international: PCT/FR2011/051607
(87) Numéro de publication internationale: WO 2012/010767

(56) Documents cités:
- WO-A1-94/18906
- WO-A2-2008/079997
- US-A- 5 262 055
- US-B1- 6 372 244

## Description

### DOMAINE DE L'INVENTION

L'invention concerne une poche pour former un organe artificiel implantable tel qu'un pancréas.

### TECHNIQUE ANTÉRIEURE

Les premières tentatives pour réaliser un organe artificiel implantable chez l'homme ou l'animal ont déjà eu lieu il y a plusieurs dizaines d'années. L'objectif est de remplacer un organe manquant par un dispositif contenant des cellules remplissant au moins une fonction dudit organe en s'affranchissant des contraintes d'une greffe.

Il a déjà été proposé dans le document FR 2 384 504 un pancréas artificiel alimenté par un liquide corporel à traiter. Le liquide passe dans un serpentin logé dans une chambre qui contient des îlots pancréatiques. La paroi du serpentin est dans une matière qui permet les échanges de molécules de faible poids telles que l'insuline et le glucose, mais qui constitue une barrière pour les molécules plus grosses telles que les anticorps et les antigènes. Cependant, un tel dispositif n'est pas implantable.

Un organe artificiel implantable a été proposé par le document WO 94/18906. Des cellules sont contenues dans une enveloppe réalisée dans une membrane semi-perméable, laquelle enveloppe est contenue dans un autre contenant assurant une protection mécanique de la première enveloppe. Les cellules sont par exemple des cellules de thyroïde, de glandes parathyroïdes, de glande surrénale, du foie ou du pancréas. Le remplacement des cellules nécessite le remplacement complet du dispositif.

Le document EP 664 729 propose un pancréas artificiel implantable et rechargeable

Les membranes semi-perméables ont fait l'objet de nombreuses recherches. Le document WO 02/060409 propose par exemple une membrane semi-perméable pour encapsuler des cellules réalisée en polycarbonate poreux et traitée en surface avec un polymère hydrophile. Ces membranes présentent les caractéristiques attendues, à savoir un bon contrôle de la perméabilité, laissant passer rapidement les nutriments et les substances générées par les cellules de l'organe artificiel, une adhésion limitée des cellules sur la surface de la membrane pour ne pas obstruer les échanges, une bonne résistance mécanique et une imperméabilité aux molécules de grande taille. Cependant, des poches réalisées avec cette matière ne contrôlent pas la répartition des îlots à l'intérieur. Il peut se former des amas qui ont du mal à échanger alors des nutriments et des substances générées.

Une poche selon le préambule de la revendication 1 est connue du document US-A-6 372 244.

### OBJECTIFS DE L'INVENTION

L'invention vise à fournir une poche pour former un organe artificiel implantable qui garantisse une bonne répartition des cellules actives et qui soit résistante pendant et après l'implantation de la poche.

### EXPOSÉ DE L'INVENTION

Avec ces objectifs en vue, l'invention a pour objet une poche pour former un organe artificiel implantable comportant une enveloppe fermée et réalisée dans une membrane semi-perméable, caractérisée en ce qu'elle comporte une nappe contenue dans l'enveloppe, la nappe comportant à sa surface des protubérances afin de maintenir un espace pour des cellules entre la nappe et l'enveloppe, la nappe et les protubérances étant d'un seul tenant et en élastomère.

Les cellules de l'organe artificiel peuvent ainsi se loger entre les protubérances sur toute la surface de la nappe sans être comprimées par l'enveloppe contre la nappe. On constate que les cellules ne forment pas d'amas et conservent une grande surface d'échange, ce qui garantit leur pérennité.

La nappe comporte par exemple des protubérances sur ses deux faces.

Selon une réalisation particulière, les protubérances ont la forme de tirets espacés les uns des autres et constituant des lignes régulièrement réparties parallèlement les unes aux autres. Cette disposition définit des canaux dans la direction desquels les fluides peuvent circuler facilement, des canaux étant orientés pour certains dans la direction des tirets, et pour d'autres de manière oblique par rapport auxdits tirets.

Selon une autre caractéristique, la poche comporte au moins un connecteur comportant un corps fixé sur la nappe, et un conduit relié au connecteur pour être en communication hydraulique avec l'intérieur de la poche. Il est ainsi possible de remplir ou de vider la poche. La fixation du connecteur sur la nappe permet que l'enveloppe soit préservée des efforts qui pourraient survenir entre la poche et le conduit, du fait que les efforts sont repris par la nappe. La nappe peut être renforcée mécaniquement, tandis que l'enveloppe conserve sa finesse utile pour assurer sa semi-perméabilité.

Selon un perfectionnement, la poche comporte en outre une chambre implantable percutanée reliée au conduit pour que la chambre implantable percutanée soit en communication hydraulique avec l'intérieur de la poche. La chambre implantable percutanée est un réceptacle fermé placé sous la peau d'une personne ou d'un animal et qui est accessible par une aiguille à travers la peau et un septum de la chambre. Ainsi, le contenu de la poche est renouvelable sans que la peau ne soit traversée en temps normal par un conduit.

De manière particulière, le connecteur comporte en outre une base, la nappe étant pincée entre la base et le corps pour fixer le connecteur sur la nappe. Le pincement permet d'obtenir une liaison mécanique qui ne fragilise pas la nappe au niveau de la jonction.

La poche comporte de préférence au moins deux connecteurs dont l'un comporte une grille interposée dans le passage hydraulique. On peut ainsi établir une circulation entre les deux connecteurs afin d'assurer un renouvellement du contenu de la poche. La présence d'une grille permet de retenir les cellules si on souhaite les maintenir dans la poche. Pour cela, le connecteur à grille est utilisé pour l'aspiration. Si on souhaite un renouvellement des cellules, on établit une circulation dans le sens inverse pour que les cellules soient évacuées par l'intermédiaire du connecteur sans grille.

Selon une disposition constructive, le connecteur comporte un chapeau, une membrane supérieure de l'enveloppe étant pincée entre le corps et le chapeau pour que le connecteur traverse la membrane supérieure de manière étanche. Ici également, la technique du pincement permet une traversée de l'enveloppe sans risque de détérioration de celle-ci.

La nappe est par exemple en silicone. Cette matière a de bonnes propriétés de souplesse, de résistance à l'extension et de réception des cellules à contenir dans la poche.

Selon un perfectionnement, la nappe en silicone a un traitement de surface du type SI-HPMC-CMC. SI désigne du silicone, HPMC désigne l'hydroxy-propyl-méthyl-cellulose et CMC désigne la cellulose méthylique carboxylique. Ce traitement permet d'accroître les propriétés de bio-compatibilité de la poche.

Selon un autre perfectionnement, la nappe comporte une âme de renfort en textile. Cette âme est par exemple un tissu de polyester. Elle permet de contrôler l'allongement possible de la nappe sous contrainte, en particulier pour les contraintes transmises par les connecteurs.

Selon d'autres caractéristiques, l'enveloppe est formée de deux membranes thermosoudées entre elles. La méthode de formation de l'enveloppe est simple et permet d'enfermer la nappe dans l'enveloppe. La poche comporte éventuellement un cadre en silicone recouvrant la soudure. Le cadre évite que les bords thermosoudés ne soient agressifs pour les tissus entourant la poche.

Selon un autre perfectionnement, la poche comporte en outre une sur-enveloppe perméable entourant l'enveloppe. On réalise ainsi une protection de l'enveloppe en matière semi-perméable sans limiter les échanges entre les tissus entourant la poche et l'intérieur de celle-ci. Cette protection est particulièrement utile pendant la phase d'implantation pendant laquelle les risques de déchirement sont importants.

### BRÈVE DESCRIPTION DES FIGURES

L'invention sera mieux comprise et d'autres particularités et avantages apparaîtront à la lecture de la description qui va suivre, la description faisant référence aux dessins annexés parmi lesquels :
- la figure 1 est une vue en perspective d'une poche conforme à l'invention ;
- la figure 2 est une vue en coupe, en perspective et en éclaté de la poche de la figure 1 au niveau d'un connecteur ;
- la figure 3 est une vue partielle en perspective de la poche au niveau d'un connecteur et représentant la nappe et la base d'un connecteur ;
- la figure 4 est une vue en coupe du connecteur de la poche de la figure 1 en position assemblé.

### DESCRIPTION DETAILLÉE

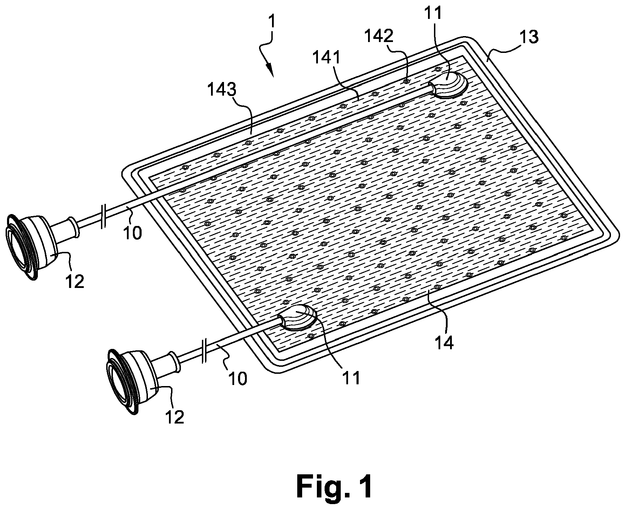

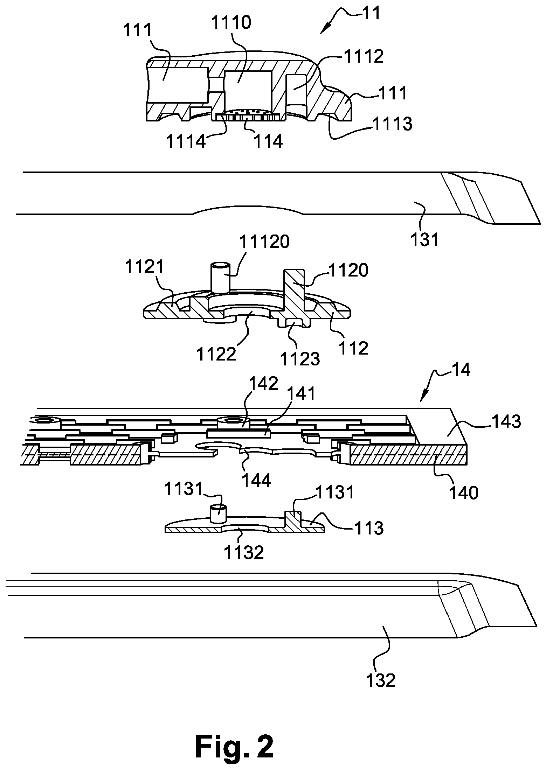

Une poche 1 conforme à l'invention, et représentée sur les figures 1 à 4, a une forme générale rectangulaire plane. Comme le montre la figure 1, la poche 1 comporte deux conduits 10 s'étendant à partir de deux connecteurs 11 jusqu'à deux chambres implantables percutanées 12, connues en soi.

La poche 1 comporte une enveloppe 13 enserrant une nappe 14. L'enveloppe 13 est formée de deux membranes 131, 132 en matière thermoplastique soudées ensemble le long de leurs bords. Les dimensions de la nappe 14 sont ajustées pour qu'elle soit contenue dans l'enveloppe 13 en étant à plat. La surface de la nappe 14 est par exemple de 50 à 200 cm².

La nappe 14 est réalisée par moulage d'une matière élastomère à base de silicone. Elle comporte une âme 140 en textile de polyester qui est surmoulée. Elle comporte sur ses deux faces des protubérances 141 qui ont la forme de tirets espacés les uns des autres et constituant des lignes régulièrement réparties parallèlement les unes aux autres. En dehors des protubérances 141, la nappe 14 a une épaisseur comprise entre 0,2 à 0,6 mm. Les tirets 141 ont par exemple une longueur de 1 à 5 mm, et les lignes sont espacées d'une distance de 1 à 2 mm. L'intervalle entre les tirets est pas exemple de 1 à 2 mm. Localement, les tirets sont renforcés par des anneaux 142 de diamètre environ 1 mm. La hauteur des protubérances 141 est par exemple de 0,2 à 0,8 mm. La périphérie de la nappe 14 comporte un bourrelet 143 de même hauteur que les protubérances 141. La surface de la nappe 14 est traitée par un revêtement SI-HPMC-CMC qui diminue la tension superficielle de la nappe 14 afin de diminuer l'adhérence des cellules sur la nappe 14 et de diminuer la sécrétion de médiateurs pro-inflammatoires.

Les deux membranes 131, 132 réalisant l'enveloppe 13 sont semi-perméables pour permettre le transfert de molécules de faible taille mais arrêter les molécules de grande taille, telles que par exemple des membranes en polycarbonate telle que décrites dans le document WO 02/060409. Les deux membranes 131, 132 sont thermosoudées à leur périphérie pour former l'enveloppe 13.

Un cadre 130 réalisé en silicone a une section en forme de U et entoure la périphérie de l'enveloppe 13 afin de recouvrir la soudure des deux membranes 131, 132.

Une communication hydraulique est réalisée entre les chambres implantables percutanées 12 par les deux conduits 10 en passant par l'intérieur de l'enveloppe 13. Pour cela, chaque conduit 10 est relié à un connecteur 11 qui réalise le passage entre l'intérieur et l'extérieur de l'enveloppe 13.

Chaque connecteur 11 comporte un chapeau 111, un corps 112 et une base 113. L'un des deux connecteurs 11 comporte en outre une grille 114 de filtration. Le chapeau 111 comporte une cavité centrale 1110 reliée à un manchon 1111 qui reçoit le conduit 10. Le conduit 10 est par exemple collé à l'intérieur du manchon 1111. Le corps 112 a une forme annulaire et comporte trois tétons de corps 1120 112 faisant saillie vers le chapeau 111. Le chapeau 111 comporte trois trous 1112 en regard des tétons de corps 1120 afin de réaliser un assemblage entre le corps 112 et le chapeau 111 par emboîtement des tétons de corps 1120 dans les trous 1112. Le corps 112 comporte en outre un renflement 1121 annulaire en correspondance avec un renfoncement 1113 de forme complémentaire réalisé dans le chapeau 111, afin de pincer et retenir l'une des membranes 131 dite supérieure entre le chapeau 111 et le corps 112. A cet endroit, la membrane supérieure 131 est percée afin de laisser passer les tétons de corps 1120. L'ouverture centrale 1122 du corps 112 est en regard de la cavité centrale 1110 du chapeau 111 afin d'établir une communication hydraulique entre eux. Dans le cas de la présence d'une grille 114, cette dernière se loge dans un épaulement 1114 du chapeau 111, au débouché de la cavité centrale 1110 vers le corps 112.

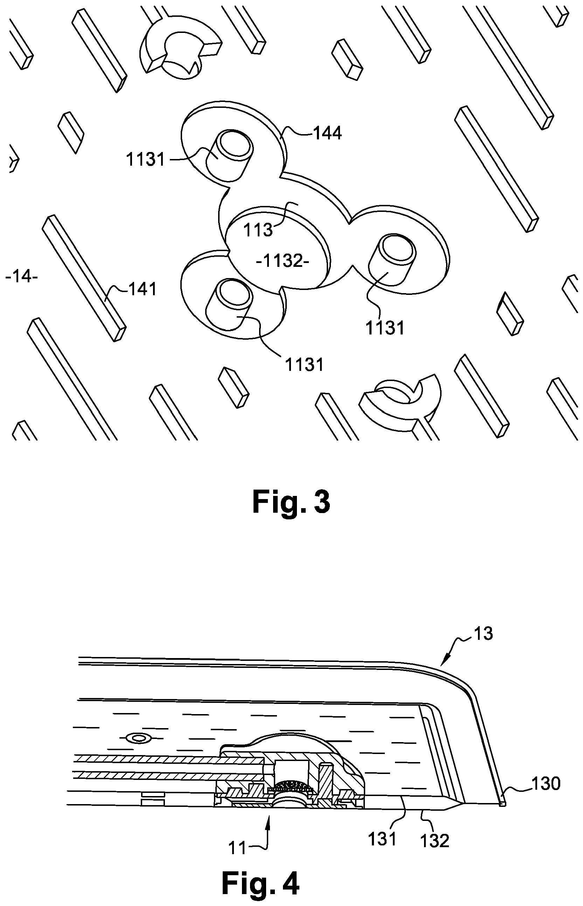

La base 113 a également une forme annulaire plane et comporte trois tétons de base 1131 faisant saillie vers le corps 112 pour s'emboîter dans des trous 1123 correspondants du corps 112. Elle permet de pincer la nappe 14 entre la base 113 et le corps 112. Pour cela, la nappe 14 comporte une découpe 144 correspondant aux trois tétons de base 113 et à l'ouverture centrale 1122 du corps 112. Les protubérances 141 sont aussi interrompues pour permettre un appui plan de la zone de pincement entre le corps 112 et la base 113, comme le montre la figure 3. L'ouverture centrale 1132 de la base 113 est aussi en regard de la cavité centrale 1110 du chapeau 111 afin d'établir une communication hydraulique entre eux.

Les tétons 1120, 1131 reçoivent par exemple de la colle afin de réaliser l'assemblage permanent des pièces 111, 112, 113 entre elles. Dans un autre mode de réalisation, l'emmanchement peut être forcé ou conique, ou les tétons peuvent être soudés par ultrasons.

Les composants 111, 112, 113 du connecteur 11 sont réalisés par injection plastique de polypropylène. Ils reçoivent éventuellement un traitement de surface afin d'augmenter la biocompatibilité.

L'assemblage de la poche 1 est réalisé de la manière suivante. La base 113 de chaque connecteur 11 est placée sous la nappe 14. Le corps 112 de chaque connecteur 11 est placé par dessus la nappe 14 et assemblé avec la base 113 correspondante en pinçant la nappe 14. Les membranes 131, 132 sont mises en place par-dessous et par-dessus la nappe 14, puis elles sont soudées ensemble sur les bords. Le cadre 130 est mis en place par collage pour recouvrir ces soudures. Le chapeau 111 des connecteurs 11 est placé par dessus la membrane supérieure 131 et assemblé avec le corps 112 correspondant en pinçant la membrane supérieure 131 entre le renfoncement 1113 et le renflement 1121. Le cas échéant, la grille 114 est placée dans l'épaulement 1114 du chapeau 111 avant l'assemblage avec le corps 112. Les conduits 10 sont connectés aux chapeaux 111 et aux chambres implantables percutanées 12.

Lors de l'utilisation de la poche 1, celle-ci est roulée sur elle-même si nécessaire et introduite dans un corps par une incision de faible taille, puis déroulée. Les chambres implantables percutanées 12 sont également introduites et placés sous la peau. Lorsqu'on est certain que la poche 1 est bien tolérée par l'organisme receveur, on introduit les cellules avec une seringue par la chambre implantable percutanée 12 reliée au connecteur 11 sans grille. Les éléments introduits passent dans la chambre implantable percutanée 12, puis dans le conduit 10, dans la cavité centrale 1110 du chapeau 111, à travers les ouvertures de la membrane et du corps 112, puis à travers la découpe 144 de la nappe 14. Les cellules se répartissent dans la poche 1 et se logent entre les protubérances 141. Éventuellement, on établit une circulation de liquide par aspiration à travers l'autre chambre implantable percutanée 12.

L'invention n'est pas limitée à l'exemple qui vient d'être décrit. La poche pourrait être en forme de disque ou de forme plane quelconque. Les protubérances pourraient avoir une forme de picots, de bosses, d'anneaux ou de cônes. Les connecteurs 11 peuvent être réalisés en d'autres matières biocompatibles telles que du polysulfone ou du polycarbonate. Une surenveloppe perméable peut entourer l'enveloppe. Le cadre 130 n'est pas indispensable, en particulier avec la présence de la surenveloppe.

## Revendications

1. Poche pour former un organe artificiel implantable comportant une enveloppe (13) fermée et réalisée dans une membrane (131, 132) semi-perméable, **caractérisée en ce qu'**elle comporte une nappe (14) contenue dans l'enveloppe (13), la nappe (14) comportant à sa surface des protubérances (141) afin de maintenir un espace pour des cellules entre la nappe (14) et l'enveloppe (13), la nappe (14) et les protubérances (141) étant réalisées d'un seul tenant par moulage d'une matière élastomère.

2. Poche selon la revendication 1, dans laquelle la nappe (14) comporte des protubérances (141) sur ses deux faces.

3. Poche selon la revendication 2, dans laquelle les protubérances (141) ont la forme de tirets espacés les uns des autres et constituant des lignes régulièrement réparties parallèlement les unes aux autres.

4. Poche selon la revendication 1, **caractérisée en ce qu'**elle comporte au moins un connecteur (11) comportant un corps (112) fixé sur la nappe (14), et un conduit (10) relié au connecteur (11) pour être en communication hydraulique avec l'intérieur de la poche (1).

5. Poche selon la revendication 4, **caractérisée en ce qu'**elle comporte en outre une chambre implantable percutanée (12) relié au conduit (10) pour que la chambre implantable percutanée (12) soit en communication hydraulique avec l'intérieur de la poche (1).

6. Poche selon la revendication 4, dans laquelle le connecteur (11) comporte en outre une base (113), la nappe (14) étant pincée entre la base (113) et le corps (112) pour fixer le connecteur (11) sur la nappe (14).

7. Poche selon la revendication 4, **caractérisée en ce qu'**elle comporte au moins deux connecteurs (11) dont l'un comporte une grille (114) interposée dans le passage hydraulique.

8. Poche selon la revendication 4, dans laquelle le connecteur (11) comporte un chapeau (111), une membrane supérieure (131) de l'enveloppe (13) étant pincée entre le corps (112) et le chapeau (111) pour que le connecteur (11) traverse la membrane supérieure (131) de manière étanche.

9. Poche selon la revendication 1, dans laquelle la nappe (14) est en silicone.

10. Poche selon la revendication 9, dans laquelle la nappe (14) en silicone a un traitement de surface du type SI-HPMC-CMC.

11. Poche selon la revendication 1, dans laquelle la nappe (14) comporte une âme (140) de renfort en textile.

12. Poche selon la revendication 1, dans laquelle l'enveloppe (13) est formée de deux membranes (131, 132) thermosoudées entre elles.

13. Poche selon la revendication 12, dans laquelle la poche comporte un cadre (130) en silicone recouvrant la soudure.

14. Poche selon la revendication 1, **caractérisée en ce qu'**elle comporte en outre une sur-enveloppe perméable entourant l'enveloppe (13).

## Patentansprüche

1. Beutel zum Formen eines implantierbaren künstlichen Organs, aufweisend eine geschlossene Hülle (13), die aus einer halbdurchlässigen Membran (131, 132) hergestellt ist, **dadurch gekennzeichnet, dass** er eine in der Hülle (13) enthaltene Lage (14) aufweist, wobei die Lage (14) auf ihrer Oberfläche Vorsprünge (141) aufweist, um einen Raum für Zellen zwischen der Lage (14) und der Hülle (13) bereitzuhalten, wobei die Lage (14) und die Vorsprünge (141) einstückig durch Formen eines Elastomermaterials hergestellt sind.

2. Beutel nach Anspruch 1, wobei die Lage (14) Vorsprünge (141) auf ihren zwei Seiten aufweist.

3. Beutel nach Anspruch 2, wobei die Vorsprünge (141) die Form von Strichen haben, die voneinander beabstandet sind und gleichmäßig zueinander parallel verteilte Linien darstellen.

4. Beutel nach Anspruch 1, **dadurch gekennzeichnet, dass** er mindestens ein Verbindungsstück (11) aufweist, das einen auf der Lage (14) befestigten Körper (112) und eine Leitung (10) aufweist, die mit dem Verbindungsstück (11) verbunden ist, um mit dem Innern des Beutels (1) in hydraulischer Kommunikation zu sein.

5. Beutel nach Anspruch 4, **dadurch gekennzeichnet, dass** er ferner eine durchbrochene implantierbare Kammer (12) aufweist, die mit der Leitung (10) verbunden ist, damit die durchbrochene implantierbare Kammer (12) in hydraulischer Kommunikation mit dem Innern des Beutels (1) ist.

6. Beutel nach Anspruch 4, wobei das Verbindungsstück (11) ferner eine Basis (113) aufweist, wobei die Lage (14) zwischen der Basis (113) und dem Körper (112) geklemmt ist, um das Verbindungsstück (11) auf der Lage (14) zu befestigen.

7. Beutel nach Anspruch 4, **dadurch gekennzeichnet, dass** er mindestens zwei Verbindungsstücke (11) aufweist, von dem eines ein in den Hydraulikdurchgang zwischengestelltes Gitter (114) aufweist.

8. Beutel nach Anspruch 4, wobei das Verbindungsstück (11) eine Kappe (111) aufweist, wobei eine obere Membran (131) der Hülle (13) zwischen dem Körper (112) und der Kappe (111) geklemmt ist, damit das Verbindungsstück (11) die obere Membran (131) dicht durchquert.

9. Beutel nach Anspruch 1, wobei die Lage (14) aus Silikon ist.

10. Beutel nach Anspruch 9, wobei die Lage (14) aus Silikon eine Oberflächenbehandlung vom Typ SI-HPMC-CMC hat.

11. Beutel nach Anspruch 1, wobei die Lage (14) einen verstärkenden Kern (140) aus Textil aufweist.

12. Beutel nach Anspruch 1, wobei die Hülle (13) aus zwei miteinander thermisch verschweißten Membranen (131, 132) gebildet ist.

13. Beutel nach Anspruch 12, wobei der Beutel einen Rahmen (130) aus Silikon aufweist, welcher die Schweißnaht bedeckt.

14. Beutel nach Anspruch 1, **dadurch gekennzeichnet, dass** er ferner eine durchlässige Umhüllung aufweist, die die Hülle (13) umgibt.

## Claims

1. Pouch for forming an implantable artificial organ comprising a closed envelope (13) made of a semi-permeable membrane (131, 132), **characterized in that** it comprises a sheet (14) contained in the envelope (13), the sheet (14) comprising, at its surface, protuberances (141) so as to maintain a space for cells between the sheet (14) and the envelope (13), the sheet (14) and the protuberances (141) being integrally produced by molding an elastomer material.

2. Pouch according to claim 1, wherein the sheet (14) comprises protuberances (141) on both of its faces.

3. Pouch according to claim 2, wherein the protuberances (141) have the shape of dashes spaced apart from one another and forming regularly distributed lines parallel to one another.

4. Pouch according to claim 1, **characterized in that** it comprises at least one connector (11) comprising a body (112) attached to the sheet (14), and a conduit (10) connected to the connector (11) so as to be in hydraulic communication with the inside of the pouch (1).

5. Pouch according to claim 4, **characterized in that** it further comprises an implantable percutaneous chamber (12) connected to the conduit (10) so that the implantable percutaneous chamber (12) is in hydraulic communication with the inside of the pouch (1).

6. Pouch according to claim 4, wherein the connector (11) further comprises a base (113), the sheet (14) being clamped between the base (113) and the body (112) in order to attach the connector (11) to the sheet (14).

7. Pouch according to claim 4, **characterized in that** it comprises at least two connectors (11), one of which comprises a grid (114) interposed in the hydraulic passage.

8. Pouch according to claim 4, wherein the connector (11) comprises a cap (111), an upper membrane (131) of the envelope (13) being clamped between the body (112) and the cap (111) so that the connector (11) passes tightly through the upper membrane (131).

9. Pouch according to claim 1, wherein the sheet (14) is made of silicone.

10. Pouch according to claim 9, wherein the silicone sheet (14) has a surface treatment of the SI-HPMC-CMC type.

11. Pouch according to claim 1, wherein the sheet (14) comprises a textile reinforcement core (140).

12. Pouch according to claim 1, wherein the envelope (13) is formed by two membranes (131, 132) heat-sealed together.

13. Pouch according to claim 12, wherein the pouch comprises a silicone frame (130) covering the seam.

14. Pouch according to claim 1, **characterized in that** it further comprises a permeable over-envelope surrounding the envelope (13).
